# EUROPEAN PATENT APPLICATION

(11) **EP 4 589 010 A1**
(43) Date of publication of application: **23.07.2025**
(21) Application number: 23865464.4
(22) Date of filing: 11.09.2023
(51) Int. Cl.: C12Q 1/06, C07K 14/705, C12N 5/0793, C12N 5/10, C12N 15/12, G01N 33/15, G01N 33/50

(54) **SCREENING METHOD FOR OFFENSIVE ODOR SUPPRESSING MATERIAL**

(30) Priority: 12.09.2022 JP 2022144633
(71) Applicant: Takasago International Corporation, Tokyo 144-8721 (JP)
(72) Inventor: MURAI Masato, Hiratsuka-shi, Kanagawa 254-0073 (JP); KASHIWAGI Takahiro, Hiratsuka-shi, Kanagawa 254-0073 (JP)
(74) Representative: Hoffmann Eitle
(86) International application number: PCT/JP2023/033021
(87) International publication number: WO 2024/058111

(57) **Abstract**

The present invention provides a method or the like for efficiently screening candidate substances for a hexanal-odor suppressing material. A screening method according to the present invention comprises: adding a test substance and hexanal to a specific olfactory receptor polypeptide; measuring a response of the olfactory receptor polypeptide with respect to the hexanal; and identifying, on the basis of the measured response, that the test substance suppressing a response of the olfactory receptor polypeptide is a candidate substance for a hexanal-odor suppressing material.

## Description

### TECHNICAL FIELD

The present invention relates to a method for screening a candidate substance for an offensive odor-suppressing material.

### BACKGROUND ART

In recent years, more and more people have become sensitive to odors and offensive odors around them in response to the diversification of consumers' palatability and living environments. The range of unpleasant odors varies from smell in living spaces to slight peculiar odor (off-flavors) in foods, and technologies that more effectively reduce and eliminate unpleasant odors are required to improve the quality of life.

Representative causative odors of unpleasant odors in living spaces include aldehyde odors such as hexanal contained in body odor, half-dried laundry odor, bathroom odor, and nursing care odor (Patent literature 1-3 and Non-patent literature 1). In addition, aldehyde odors, one of the typical odors found in the living room of an average home, are considered to be an important target for odor elimination because they are considered to be an odor group that contributes significantly to the odors in the living room (Non-patent literature 2). In general, aldehydes such as hexanal contained in body odor are said to be derived from unsaturated fatty acids, and are generated by peroxidation reactions and decomposition by indigenous microorganisms on the body surface and enzymes such as lipoxygenase in *in vivo* metabolism. Especially in the fragrance industry, hexanal is an important unpleasant odor component used to reproduce the odor of sweat, axillary odor, and scalp odor (Patent literature 4 and 5). Furthermore, aldehydes such as hexanal are not only generated by the decomposition and metabolism of sebum shed from the human body and oil used for cooking by indigenous microorganisms in living spaces, but are also naturally generated by oxidation reactions with oxygen in the air (Non-patent literature 2).

Thus, it is difficult to completely suppress the generation of aldehydes such as hexanal, which are generated naturally in living spaces due to metabolism and oxidation reactions, and there is a wide demand for odor control (masking) technology that selectively makes unpleasant odors in living spaces less noticeable through the use of fragrances.

Hexanal is also described as a "grassy smell" and is known as an off-flavor produced during the deterioration of soybeans and dairy products, contributing to reduced food palatability (Non-patent literature 3 and 4). In soybeans, hydroperoxide lyase acts on lipid peroxides generated by the action of soybean lipoxygenase on unsaturated fatty acids to produce aldehydes such as n-hexanal, which causes the grassy smell. In dairy products, linoleic acid contained in dairy fat undergoes enzymatic reactions and photo-oxidation during processing and storage of raw milk, resulting in the generation of aldehydes such as n-hexanal, which causes an unusual odor. Therefore, it is difficult to completely eliminate the generation of hexanal in foods as long as they contain fatty acids, and it is important to search for fragrance materials that can specifically eliminate the hexanal off-flavor that is perceived when eating or drinking.

Thus, the search for a fragrance material that makes hexanal odor less perceptible is very important for improving the quality of life from the aspects of food, clothing, and shelter. However, conventional methods for searching for unpleasant odor-suppressing/deodorizing substances required odor assessment specialists to confirm a large number of candidate substances for offensive odor-suppressing substances one by one by sensory assessment, resulting in a low throughput problem. On the other hand, a recently established molecular biological method can rapidly identify offensive odor-suppressing materials by searching for olfactory receptors that respond to offensive odor-causing substances and screening offensive odor-suppressing materials that suppress the response of these olfactory receptors all at once *in vitro.*

There are about 400 types of human olfactory receptors, and a single odorant is known to respond to many olfactory receptors, and some of the responsive receptors are often receptors called broadly tuned olfactory receptors, which recognize a wide range of odorants without distinction. Non-patent literature 5 mentions and gives examples of broadly tuned olfactory receptors such as OR1A1 and OR2W1 that respond widely regardless of notes. Patent literature 6 discloses that broadly tuned olfactory receptors OR1A1, OR2W1, and OR10A6 are responsible for the modulation of odor sensitivity, and that odor sensitivity can be enhanced or suppressed by enhancing or suppressing these olfactory receptor responses.

Non-patent literature 6 discloses broadly tuned olfactory receptor OR2W1 as an olfactory receptor involved in sensing hexanal, which causes the grassy smell of soybeans, and identifies alpha-pinene, etc. as masking agents that suppress the OR2W1 response.

Accordingly, although there are olfactory receptors that are known to respond to hexanal, broadly tuned olfactory receptor OR2W1 is the only olfactory receptor that has shown a concentration-dependent hexanal response in *in vitro* tests and that is actually associated with the recognition of hexanal odors.

### CITATION LIST

### Patent Literature

Patent literature 1: Japanese Unexamined Patent Application Publication No. H11-286423
Patent literature 2: Japanese Patent No. 4950927
Patent literature 3: Japanese Unexamined Patent Application Publication No. 2007-14749
Patent literature 4: Japanese Unexamined Patent Application Publication No. 2004-263102
Patent literature 5: Japanese Unexamined Patent Application Publication No. 2011-184469
Patent literature 6: Japanese Unexamined Patent Application Publication No. 2018-121623
Patent literature 7: WO2019/230903
Patent literature 8: WO2021/193429

### Non-patent literature

Non-patent literature 1: Kikuya OGATA, "Designing deodorizing function" with the aim of creating comfortable spaces in nursing and care facilities, Research Report of Kyoto Municipal Institute of Industrial Technology And Culture, No. 9, pp. 73-76 (2014)
Non-patent literature 2: Yuko NAKAMURA et al., A survey on the components of room odors at homes with different living environments, Indoor Environment, Vol. 17, No. 1, pp. 1-9 (2014)
Non-patent literature 3: Food Sci. Nutr. 2020 Jan 14; 8(2): 1139-1149
Non-patent literature 4: Milk Science, Vol. 57, No. 3, 2008
Non-patent literature 5: Flavour Fragr. J., 2015, 30, 342-361
Non-patent literature 6: Yuko TERADA et al., Identification of Human Olfactory Receptor Sensing n-Hexanal, the Causative Compound of Grassy Smell in Soybean Products, Soy Protein Research, Japan, Vol. 23, pp. 159-164 (2020)
Non-patent literature 7: Christophe Verbeurgt et.al, PLoS One. 2014. 9(5): e96333 (https://doi.org/10.1371/journal.pone.0096333)

### SUMMARY OF INVENTION

### Technical Problem

Since the use of masking agents that target broadly tuned olfactory receptors, which respond to a wide range of odors and modulate odor sensitivity, indiscriminately suppress a wide range of odors, they may even mask pleasant odors in foods and fragrances, making the overall odor weaker. Under these circumstances, it is desirable to identify a group of olfactory receptors that are truly important for the recognition of hexanal odor itself, rather than broadly tuned olfactory receptors that recognize a wide range of odors without distinction, and to develop a method for efficiently screening a candidate substance for a hexanal odor-suppressing material.

### Solution to Problem

The present invention was made in consideration of the above circumstances and provides a method for screening a candidate substance for a hexanal odor-suppressing material described below.

[1] A method for screening a candidate substance for a hexanal odor-suppressing material, comprising:
   adding a test substance and hexanal to at least one olfactory receptor polypeptide selected from the group consisting of olfactory receptors OR2C1, OR2J2, OR2J3, OR5K1, OR5P3, and OR51E1, and polypeptides that contain an amino acid sequence having 80% or more identity to the amino acid sequence of any of said olfactory receptors and that show responsiveness to hexanal, which is an offensive odor-causing substance;
   measuring the response of the olfactory receptor polypeptide to hexanal; and
   identifying, based on the measured response, the test substance which suppresses the response of the olfactory receptor polypeptide as a candidate substance for a hexanal odor-suppressing material.
[2] The method according to [1], wherein the hexanal odor is an odor caused by hexanal or an odor containing at least some of said odor, which, in some cases, is an odor that is unpleasant to humans, and in some cases, is one or more of body odor, sweat odor, axillary odor, scalp odor, half-dried laundry odor, bathroom odor, nursing care odor, and deterioration odor of soybean or dairy products.
[3] The method according to [1] or [2], comprising measuring the response of the olfactory receptor polypeptide to a hexanal odor-causing substance on cells isolated from a living body expressing the olfactory receptor or on cells artificially expressing the olfactory receptor by genetic engineering.
[4] The method according to any one of [1]-[3], wherein the response of the olfactory receptor polypeptide is measured by reporter assay or calcium imaging.

### ADVANTAGEOUS EFFECTS OF INVENTION

The method of the present invention can be used to screen a candidate substance for a hexanal odor-suppressing material that can inhibit the binding of hexanal to olfactory receptors OR2C1, OR2J2, OR2J3, OR5K1, OR5P3, and OR51E1.

### BRIEF DESCRIPTION OF DRAWINGS

[Figure 1] A graph showing the results of the measurement of the response of olfactory receptor OR2C1 to hexanal.
[Figure 2] A graph showing the results of the measurement of the response of olfactory receptor OR2J2 to hexanal.
[Figure 3] A graph showing the results of the measurement of the response of olfactory receptor OR2J3 to hexanal.
[Figure 4] A graph showing the results of the measurement of the response of olfactory receptor OR5K1 to hexanal.
[Figure 5] A graph showing the results of the measurement of the response of olfactory receptor OR5P3 to hexanal.
[Figure 6] A graph showing the results of the measurement of the response of olfactory receptor OR51E1 to hexanal.
[Figure 7] A graph showing the effect of the addition of beta-caryophyllene oxide, which is a hexanal odor-suppressing material, on the suppression of the response of the olfactory receptor OR2C1 to hexanal.
[Figure 8] A graph showing the effect of the addition of menthyl lactate, which is a hexanal odor-suppressing material, on the suppression of the response of the olfactory receptor OR2J2 to hexanal.
[Figure 9] A graph showing the effect of the addition of nootkatone, which is a hexanal odor-suppressing material, on the suppression of the response of the olfactory receptor OR2J3 to hexanal.
[Figure 10] A graph showing the effect of the addition of beta-caryophyllene oxide, which is a hexanal odor-suppressing material, on the suppression of the response of the olfactory receptor OR5K1 to hexanal.
[Figure 11] A graph showing the effect of the addition of menthyl lactate, which is a hexanal odor-suppressing material, on the suppression of the response of the olfactory receptor OR5P3 to hexanal.
[Figure 12] A graph showing the effect of the addition of beta-caryophyllene oxide, which is a hexanal odor-suppressing material, on the suppression of the response of the olfactory receptor OR51E1 to hexanal.

### DESCRIPTION OF EMBODIMENTS

### Overview of the present invention

This specification incorporates the entire specification of Japanese Patent Application No. 2022-144633 (filed on September 12, 2022), based on which priority is claimed by this application. All publications cited herein, such as prior art literature, as well as published patent applications, patent publications, and other patent literature, are incorporated herein by reference.

The present invention provides a method for efficiently screening a candidate substance for a hexanal odor-suppressing material by searching for a group of olfactory receptors that truly show a concentration-dependent response to hexanal and, consequently, provides a hexanal odor-suppressing composition that reduces the unpleasantness of the hexanal odor.

The present inventors have newly found that specific receptors among the approximately 400 human olfactory receptors, namely OR2C1, OR2J2, OR2J3, OR5K1, OR5P3, and OR51E1, show a concentration-dependent response to the offensive odorant hexanal. The present inventors have also obtained a novel finding that known hexanal odor-suppressing materials, which have the ability to suppress hexanal odor as demonstrated in sensory tests by human olfaction, suppress the above specific olfactory receptors, and have identified the above specific olfactory receptor group as an olfactory receptor group that is truly important for hexanal odor recognition. Accordingly, it is possible to assess and select hexanal odor-suppressing materials by the masking effect of olfactory receptor antagonists. The present invention was found based on these novel findings.

Hereinafter, a method for screening a candidate substance for a hexanal odor-suppressing material according to the present invention (hereinafter referred to as the screening method of the present invention) will be described specifically.

As mentioned above, the screening method of the present invention comprises the following steps:
adding a test substance and hexanal to at least one olfactory receptor polypeptide selected from the group consisting of human olfactory receptors OR2C1, OR2J2, OR2J3, OR5K1, OR5P3, and OR51E1, and polypeptides that contain an amino acid sequence having 80% or more identity to the amino acid sequence of any of said olfactory receptors and that show responsiveness to hexanal;
measuring the response of the olfactory receptor polypeptide to hexanal; and
identifying, based on the measured response, the test substance which suppresses the response of the olfactory receptor polypeptide as a candidate substance for a hexanal odor-suppressing material. The present invention can be implemented, for example, according to the following embodiments.

According to one embodiment, the present invention is a method for screening a candidate substance for a hexanal odor-suppressing material from among test substances using OR2C1, OR2J2, OR2J3, OR5K1, OR5P3 and OR51E1 that show responsiveness to hexanal, which is an offensive odor-causing substance, the method characterized by comprising the steps of:
(i) bringing hexanal into contact with an olfactory receptor polypeptide selected from the group consisting of human olfactory receptors OR2C1, OR2J2, OR2J3, OR5K1, OR5P3, and OR51E1, and proteins (polypeptides) that contain an amino acid sequence having 80% or more identity to the amino acid sequence of any of said olfactory receptors and that show responsiveness to hexanal to measure the basal activity response of the olfactory receptor before the contact to hexanal (T0) and the response of the olfactory receptor to hexanal at any time after the contact to hexanal (Tx);
(ii) comparing the basal activity response of the olfactory receptor before the contact to hexanal (T0) and the response of the olfactory receptor to hexanal at any time after the contact to hexanal (Tx) measured in step (i) to calculate and determine the value of the change in responsiveness;
(iii) mixing a test substance with hexanal in step (i) and calculating and determining the value of the change in responsiveness in the same manner as in step (ii); and
(iv) selecting the test substance whose value in step (iii) is reduced compared to that in step (ii) as a candidate substance for an hexanal odor-suppressing material.

According to the screening method of the present invention, a candidate substance for a hexanal odor-suppressing material is selected from test substances by using, as an indicator, the responsiveness of the test substances to an olfactory receptor polypeptide selected from the group consisting of human olfactory receptors OR2C1, OR2J2, OR2J3, OR5K1, OR5P3, and OR51E1, and proteins (polypeptides) that contain an amino acid sequence having 80% or more identity to the amino acid sequence of any of said olfactory receptors and that show responsiveness to hexanal.

In *in vitro* olfactory receptor assays, assessments are generally performed under the assumption that all olfactory receptors are expressed at the same level, and thus the higher the response value per olfactory receptor unit, the more sensitive the olfactory receptor is considered to be. However, the expression levels of olfactory receptors actually expressed in the human olfactory epithelium vary depending on the types of the olfactory receptors, and olfactory receptors that respond strongly in *in vitro* assays do not necessarily transmit odor sensation in the olfactory neurons located in the human olfactory epithelium strongly to the central nerve system of the brain. Therefore, even if an olfactory receptor has a low response value to an odorant in an *in vitro* olfactory receptor assay, its response to odorant stimuli may be stronger in the olfactory neurons that express olfactory receptors with high expression levels in the actual human olfactory epithelium, thus contributing significantly to odor recognition. According to Non-patent literature 7 listed above, with respect to the actual expression levels of olfactory receptors in humans, the average expression level of the broadly tuned olfactory receptor OR2W1 is very low as (6±13), while the average expression levels of the hexanal olfactory receptors specified by the inventors of the present application after careful investigation are 78, 37, 82, 598, 752 and 170 for OR2C1, OR2J2, OR2J3, OR5K1, OR5P3 and OR51E1, respectively, indicating that the expression levels of all the specified olfactory receptors are generally high and contribute greatly to hexanal odor recognition in the human olfactory system. Therefore, by assessing the responsiveness of test substances using hexanal olfactory receptors OR2C1, OR2J2, OR2J3, OR5K1, OR5P3 and OR51E1 as targets, a candidate substance that can prevent hexanal from binding to the olfactory receptors can be selected among the test substances. A "test substance" as used herein refers to, but is not particularly limited to, a compound, composition or mixture subjected to an investigation for hexanal odor-suppressing effect. In addition, a "hexanal odor-suppressing material" as used herein refers to, but is not particularly limited to, a compound, composition or mixture that can suppress hexanal odor. Hereinafter, each step of the screening method of the present invention (steps (i)-(v) described above) will be described.

### <Step (i)>

In step (i), hexanal is brought into contact with an olfactory receptor polypeptide selected from the group consisting of human olfactory receptors OR2C1, OR2J2, OR2J3, OR5K1, OR5P3, and OR51E1, and proteins (polypeptides) that contain an amino acid sequence having 80% or more identity to the amino acid sequence of any of said olfactory receptors and that show responsiveness to hexanal to measure the basal activity response of the olfactory receptor before the contact to hexanal (T0) and the response of the olfactory receptor to hexanal at any time after the contact to hexanal (Tx);

An olfactory receptor selected from the group consisting of olfactory receptors OR2C1, OR2J2, OR2J3, OR5K1, OR5P3, and OR51E1, and proteins (polypeptides) that contain an amino acid sequence having 80% or more identity to the amino acid sequence of any of said olfactory receptors and that show responsiveness to hexanal is used.

OR2C1 is registered in the NCBI (GenBank) website (http://www.ncbi.nlm.nih.gov/) under "Accession number: NM_012368.2" or "Accession number: NP_036500.2" and is a protein (polypeptide) consisting of the amino acid sequence (SEQ ID NO:2) encoded by the 53rd to 991st DNAs of the nucleotide sequence represented by SEQ ID NO:1.

OR2J2 is registered in the NCBI (GenBank) website under "Accession number: NM_030905.2" or "Accession number: NP_112167.2" and is a protein (polypeptide) consisting of the amino acid sequence (SEQ ID NO:4) encoded by the 103rd to 1041st DNAs of the nucleotide sequence represented by SEQ ID NO:3.

OR2J3 is registered in the NCBI (GenBank) website under "Accession number: NM_001005216.3" or "Accession number: NP_001005216.2" and is a protein (polypeptide) consisting of the amino acid sequence (SEQ ID NO:6) encoded by the 82nd to 1017th DNAs of the nucleotide sequence represented by SEQ ID NO:5.

OR5K1 is registered in the NCBI (GenBank) website under "Accession number: NM_001004736.3" or "Accession number: NP_001004736.2" and is a protein (polypeptide) consisting of the amino acid sequence (SEQ ID NO:8) encoded by the 98th to 1024th DNAs of the nucleotide sequence represented by SEQ ID NO:7.

OR5P3 is registered in the NCBI (GenBank) website under "Accession number: NM_153445.2" or "Accession number: NP_703146.1" and is a protein (polypeptide) consisting of the amino acid sequence (SEQ ID NO:10) encoded by the 39th to 974th DNAs of the nucleotide sequence represented by SEQ ID NO:9.

OR51E1 is registered in the NCBI (GenBank) website under "Accession number: NM_152430.3" or "Accession number: NP_689643.2" and is a protein (polypeptide) consisting of the amino acid sequence (SEQ ID NO:12) encoded by the 145th to 1101st DNAs of the nucleotide sequence represented by SEQ ID NO:11.

Since the olfactory receptors OR2C1, OR2J2, OR2J3, OR5K1, OR5P3 and OR51E1 selectively respond to hexanal odor, the screening method using OR2C1, OR2J2, OR2J3, OR5K1, OR5P3 and OR51E1 can contribute to the development of a hexanal odor-suppressing material.

As an olfactory receptor, an olfactory receptor polypeptide selected from the group consisting of olfactory receptors OR2C1, OR2J2, OR2J3, OR5K1, OR5P3, and OR51E1, and proteins (polypeptides) that contain an amino acid sequence having 80% or more, preferably 85% or more, more preferably 90% or more, still more preferably 95% or more, and particularly preferably 98% or more identity to the amino acid sequence of any of said olfactory receptors and that show responsiveness to hexanal may be used. Herein, the sequence identity of the amino acid sequences is calculated by the BLAST search algorithm (publicly available from NCBI).

One type of olfactory receptor may be used alone or two or more of them may be used in combination.

According to the present invention, hexanal odor is an odor caused by hexanal or an odor containing at least some of said odor, preferably an odor that is unpleasant to humans, and examples thereof include, but are not limited to, body odor, sweat odor, axillary odor, scalp odor, half-dried laundry odor, bathroom odor, nursing care odor, and deterioration odor of soybean or dairy products.

According to the present invention, the method of bringing an olfactory receptor into contact with hexanal to measure the response of the olfactory receptor to the hexanal odor is not particularly limited. For example, the response of the olfactory receptor can be measured by bringing cells isolated from living body expressing the olfactory receptor into contact with hexanal, or the response of the olfactory receptor can be measured by bringing cells artificially expressing the olfactory receptor by genetic engineering into contact with hexanal. The time for the olfactory receptor to be in contact with hexanal depends on the concentration of hexanal and the method of measurement, so it is difficult to say, but it is 0-60 minutes, preferably 0-30 minutes in the luciferase assay according to the present invention, and it is from a few seconds to a few minutes in the calcium imaging method.

Cells artificially expressing an olfactory receptor by genetic engineering can be produced by transforming cells with vectors incorporating a gene encoding the olfactory receptor.

In a preferred aspect of the present invention, the N-terminal 20 amino acid residues of bovine rhodopsin may be incorporated along with the olfactory receptor. Incorporation of the N-terminal 20 amino acid residues of bovine rhodopsin can promote expression of the olfactory receptor in the cell membrane.

Bovine rhodopsin is registered in the NCBI (GenBank) website under "Accession number: NM_001014890.2" or "Accession number: NP_001014890.1". Bovine rhodopsin is a protein (polypeptide) consisting of the amino acid sequence (SEQ ID NO: 14) encoded by the 1st to 1047th DNAs of the nucleotide sequence represented by SEQ ID NO:13.

Instead of bovine rhodopsin, a protein (polypeptide) that contains an amino acid sequence having 80% or more, preferably 85% or more, more preferably 90% or more, still more preferably 95% or more, and particularly preferably 98% or more identity to the amino acid sequence represented by SEQ ID NO: 14 and that can promote expression of the olfactory receptor in the cell membrane may be used.

Note that amino acid residues of a protein (polypeptide) other than bovine rhodopsin may also be used as long as it can promote expression of the olfactory receptor in the cell membrane.

The method of measuring the response of the olfactory receptor is not particularly limited and any method used in this field can be used. For example, it is known that when an aroma compound binds to an olfactory receptor, it activates the intracellular G protein, which in turn activates adenylate cyclase to convert ATP to cyclic AMP (cAMP), thereby increasing the intracellular cAMP level. Therefore, the response of an olfactory receptor can be measured by measuring the cAMP level. ELISA, reporter assay, etc. is used as the method of measuring the cAMP level. Among them, it is preferable to measure the response of the olfactory receptor by reporter assay using a luminescent substance such as luciferase.

### <Step (ii)>

In step (ii), the basal activity response of the olfactory receptor before the contact to hexanal (T0) is compared with the response of the olfactory receptor to hexanal at any time after the contact to hexanal (Tx) measured in step (i) to calculate and determine the value of the change in responsiveness.

According to one embodiment of the present invention, the change in responsiveness may be assessed by using, as an indicator, the fold increase value, which is obtained by dividing the response of the olfactory receptor to hexanal at any time after the contact to hexanal (Tx) by the basial activity response of the olfactory receptor before the contact to hexanal (T0) measured in step (i). For example, when the response of the olfactory receptor is measured by reporter assay using a luminescent substance such as luciferase, the change can be assessed using an offensive odor-causing substance at a concentration at which the fold increase value is preferably 1 or more, more preferably 1.2 or more, and still more preferably 2 or more.

### <Step (iii)>

In step (iii), a test substance is mixed with hexanal in step (i) and the value of the change in responsiveness is calculated and determined as in step (ii).

### <Step (iv)>

In step (iv), the test substance whose value of the change in step (iii) is reduced compared to that in step (ii) is selected as a candidate substance for an hexanal odor-suppressing material.

In the present invention, the test substance used in step (iii) can be assessed as a candidate substance for an offensive odor-suppressing material if a reduction in the value of the change in responsiveness is observed by comparing the measurement results in steps (ii) and (iii).

In the present invention, the test substance used in step (iii) can be assessed as a candidate substance for an offensive odor-suppressing material if a reduction in the value of the change in responsiveness is observed by comparing the measurement results of the change values in steps (ii) and (iii).

In the above manner, a candidate substance for a hexanal odor-suppressing material can be screened among the test substances. According to the present invention, it is possible to select a candidate substance for a hexanal odor-suppressing material from a large number of test substances without being disturbed by problems caused by olfactory fatigue or individual differences due to sensory assessments conducted by the human sense of smell.

The selected substance can be used as a candidate substance for a hexanal odor-suppressing material. Based on the selected substance, modification or the like can be made as necessary to develop a new compound with an optimal odor. Furthermore, it is also possible to blend the selected substance with other fragrance material to develop a fragrance material that can suppress hexanal odor and that also has an optimal odor. The screening method of the present invention can be used to develop a new fragrance material as a hexanal odor-suppressing material.

### EXAMPLES

Hereinafter, the present invention will be described in more detail by means of examples, although the present invention should not be limited in any way to these examples.

### [Example 1]

Confirmation that olfactory receptors OR2C1, OR2J2, OR2J3, OR5K1, OR5P3, and OR51E1 are responsive to hexanal

### (1) Olfactory receptor gene cloning

Human olfactory receptor genes were obtained by PCR cloning from Human Genomic DNA: Female (Promega) based on the nucleotide sequence information (see SEQ ID NOS:1, 3, 5, 7, 9, and 11) registered in the NCBI (GenBank) website mentioned above. DNA encoding the N-terminal 20 amino acid residues of bovine rhodopsin (DNA consisting of the 1st to 60th nucleotides of the nucleotide sequence represented by SEQ ID NO:13) was incorporated into pME18S vector and the obtained human olfactory receptor gene was further incorporated downstream of it, thereby obtaining a human olfactory receptor gene expression vector.

### (2) Screening for expression of olfactory receptor gene responsive to hexanal

0.05 µg of human olfactory receptor gene expression vector, 0.01 µg of RTP1S vector, and 0.01 µg of firefly luciferase vector pGloSensor^{™}-22F cAMP Plasmid (Promega) containing CMV promoter were dissolved in 10 µL of Opti-MEM I (Gibco) to prepare a gene solution (per well). HEK293T cells were seeded at 100 µL per well in a 96-well plate (Biocoat, Corning) at a number of cells that reached subconfluence after 24 hours, and the gene solution was added to each well by lipofection according to the usage of Lipofectamine 3000 so that the cells were transfected with the gene and cultured at 37°C in a 5% carbon dioxide atmosphere for 24 hours. After removal of the culture solution, the cells were washed once with HEPES buffer, and 100 µL of GloSensor^{™} cAMP Reagent (Promega), adjusted to a predetermined concentration with HEPES buffer supplemented with FBS, was added to each well, and allowed to stand for 2 hours at room temperature. Then, 10 µL of 10 mM hexanal solution prepared in HEPES buffer supplemented with 300 µM CuCl₂ and FBS was added to each well, and the changes in the response value of the luciferase activity over time were measured every minute immediately after the addition, thus obtaining the responses of approximately 400 human olfactory receptors to hexanal. The magnitude of the olfactory receptor response was expressed as a fold increase value obtained by dividing the maximum response value of luciferase activity that occurred after stimulation with hexanal by the luciferase activity before the stimulation with hexanal. Accordingly, OR2C1, OR2J2, OR2J3, OR5K1, OR5P3 and OR51E1 were identified as candidate olfactory receptors responsive to hexanal. After that, an aroma compound as a sample was prepared in HEPES buffer supplemented with 300 µM CuCl₂ and FBS to a concentration 10 times the final concentration. 10 µL this aroma compound stimulation solution was added to each well, and the changes in the luciferase activity over time were measured every minute immediately after the addition to obtain the responses of approximately 400 human olfactory receptors to the aroma compound. The magnitude of the response of the olfactory receptor was expressed as a fold increase value determined by dividing the maximum luciferase activity value that occurred by the stimulation with the aroma compound by the luciferase activity before the stimulation with the aroma compound.

### (3) Identification of olfactory receptors responsive to hexanal

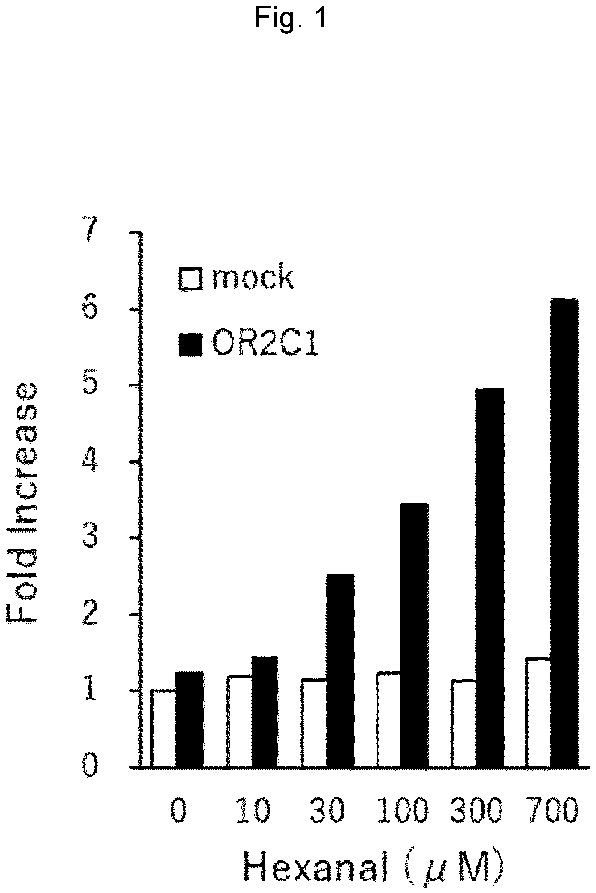

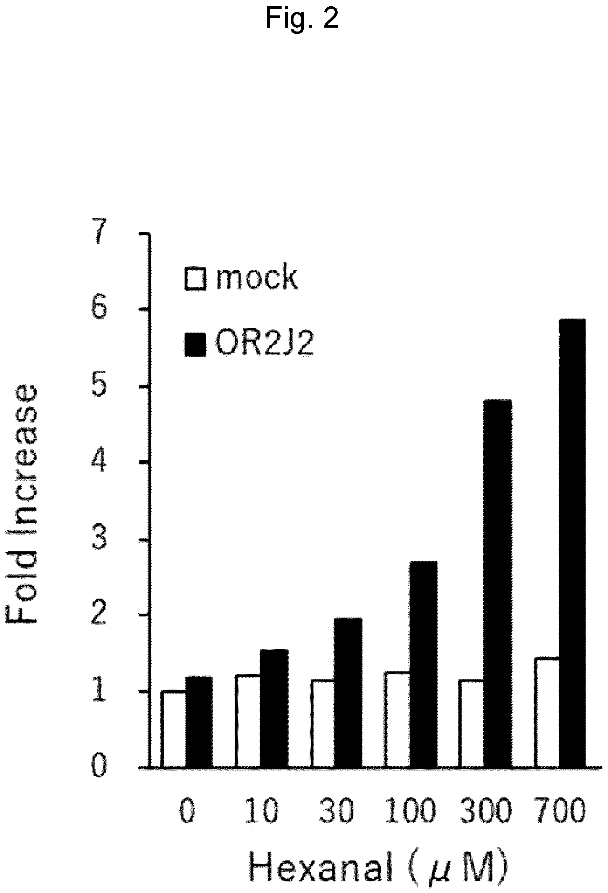

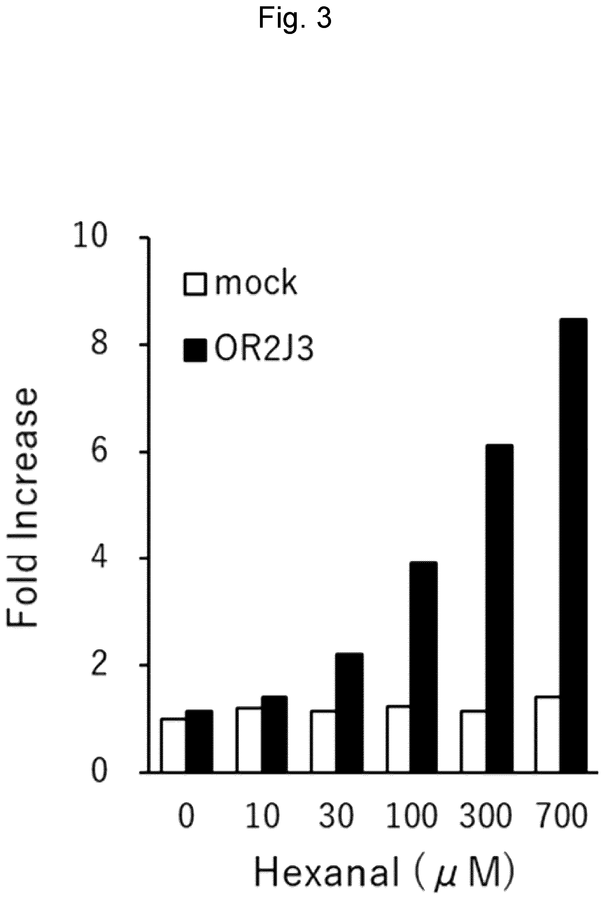

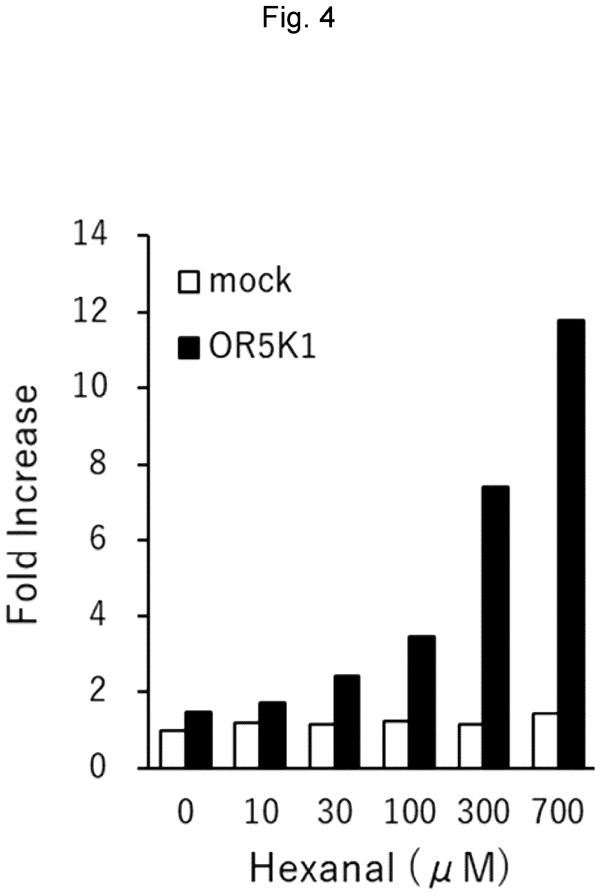

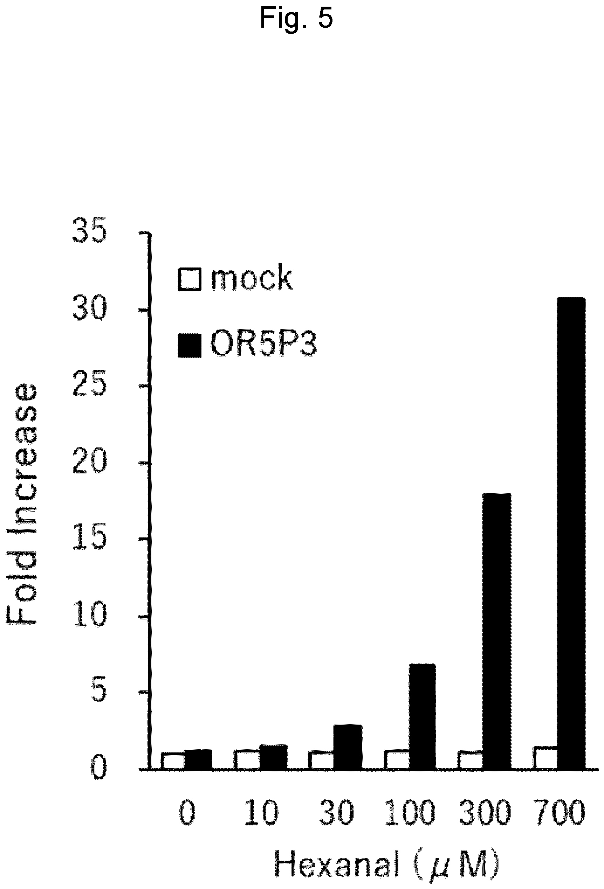

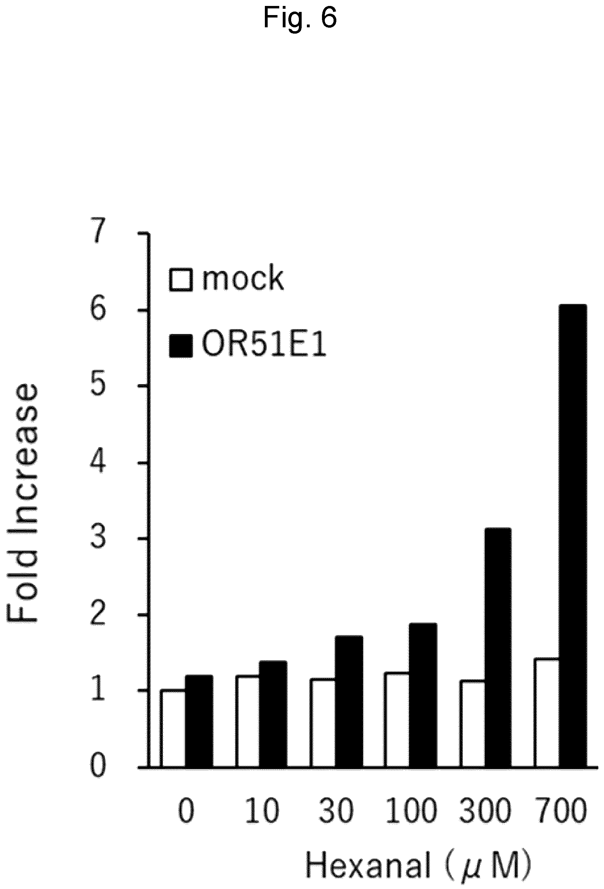

The responses of hexanal, which is an offensive odor-causing substance, to the olfactory receptors OR2C1, OR2J2, OR2J3, OR5K1, OR5P3 and OR51E1 were measured at various concentrations by the luciferase activity assay described above. The results are shown in Figures 1-6, respectively. OR2C1, OR2J2, OR2J3, OR5K1, OR5P3, and OR51E1, showed concentration-dependent responses to the different hexanal concentrations. On the other hand, no response was observed in the mock test (using cells that do not express olfactory receptors). Accordingly, OR2C1, OR2J2, OR2J3, OR5K1, OR5P3 and OR51E1 were shown to respond specifically to hexanal.

### [Example 2]

### Assessment of effects of hexanal odor-suppressing materials on suppression of responses of OR2C1, OR2J2, OR2J3, OR5K1, OR5P3, and ORS1E1

For the hexanal odor-suppressing materials that are known to mask hexanal odor in Patent literature 7 and 8 above: specifically, menthyl lactate, nootkatone, and beta-caryophyllene oxide, their effects on suppressing the responses of hexanal olfactory receptors OR2C1, OR2J2, OR2J3, OR5K1, OR5P3, and OR51E1 were determined by the following luciferase assay.

### (1) Luciferase assay

0.05 µg of gene expression vector of each of human olfactory receptors OR2C1, OR2J2, OR2J3, OR5K1, OR5P3, and OR51E1, 0.01 µg of RTP1S vector, and 0.01 µg of firefly luciferase vector pGloSensor^{™}-22F cAMP Plasmid (Promega) containing CMV promoter were dissolved in 10 µL of Opti-MEM I (Gibco) to prepare a gene solution (per well). HEK293T cells were seeded at 100 µL per well in a 96-well plate (Biocoat, Corning) at a number of cells that reached subconfluence after 24 hours, and the gene solution was added to each well by lipofection according to the usage of Lipofectamine 3000 so that the cells were transfected with the gene and cultured at 37°C in a 5% carbon dioxide atmosphere for 24 hours. After removal of the culture solution, the cells were washed once with HEPES buffer, and 100 µL of GloSensor^{™} cAMP Reagent (Promega), adjusted to a predetermined concentration with HEPES buffer supplemented with FBS, was added to each well, and allowed to stand for 2 hours at room temperature. Then, 10 µL of a solution obtained by mixing a hexanal odor-suppressing material at 3 mM in a 7 mM hexanal solution prepared in HEPES buffer supplemented with 300 µM CuCl₂ and FBS was added to each well, and the changes in the luciferase activity over time were measured every minute immediately after the addition, thus obtaining the responses of each of the human olfactory receptors over time. The magnitude of the response of the olfactory receptor was expressed as a fold increase value determined by dividing the luciferase activity that occurred by stimulation with an aroma compound by the luciferase activity before the stimulation with the aroma compound.

### (2) Assessment of hexanal odor-suppressing materials on suppression of olfactory receptor responses

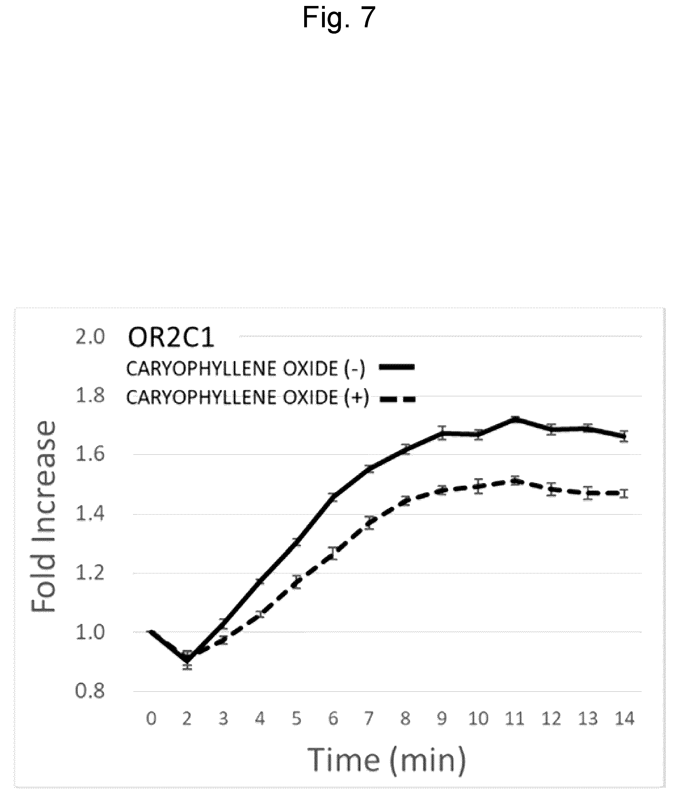

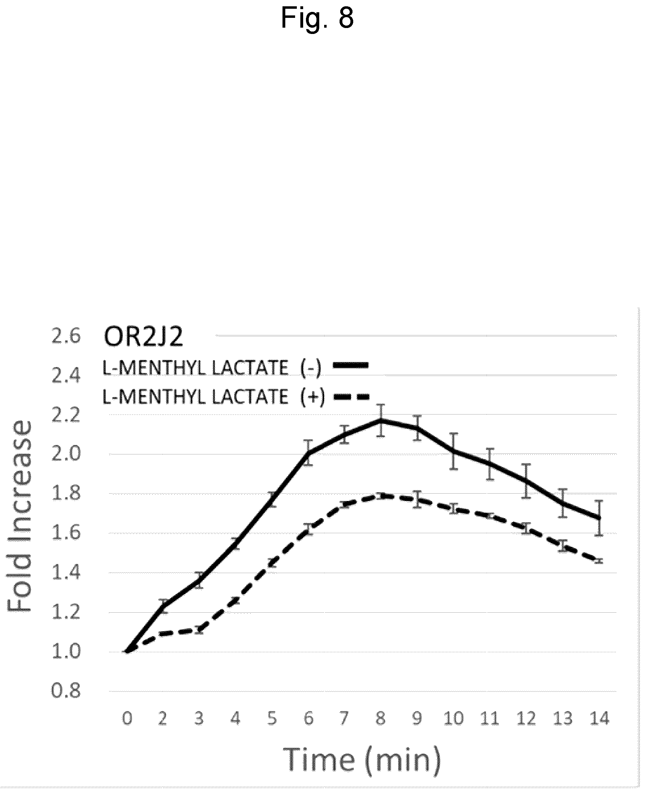

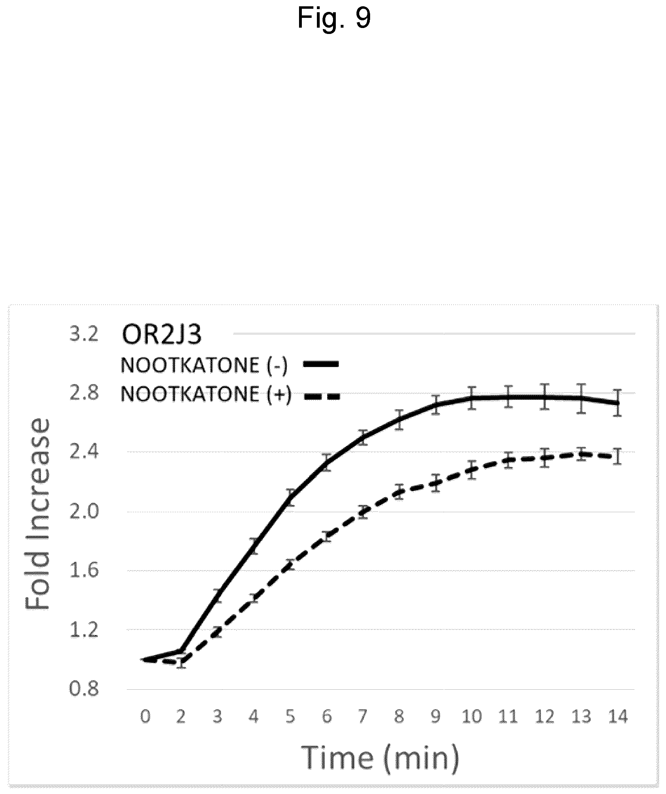

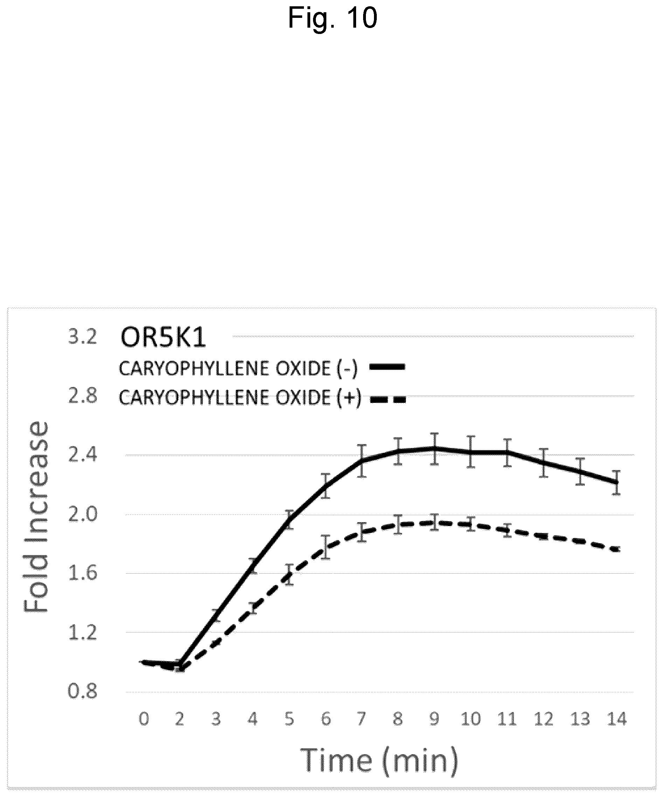

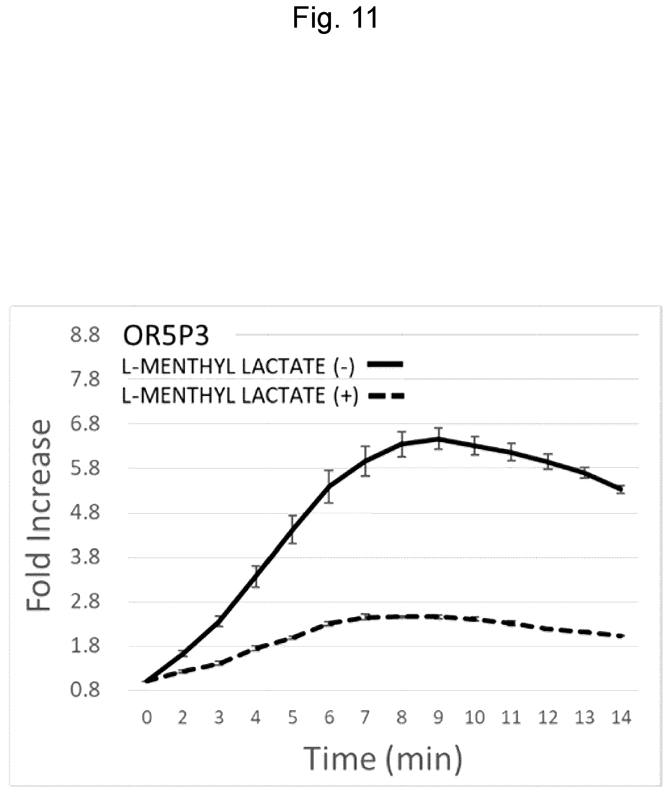

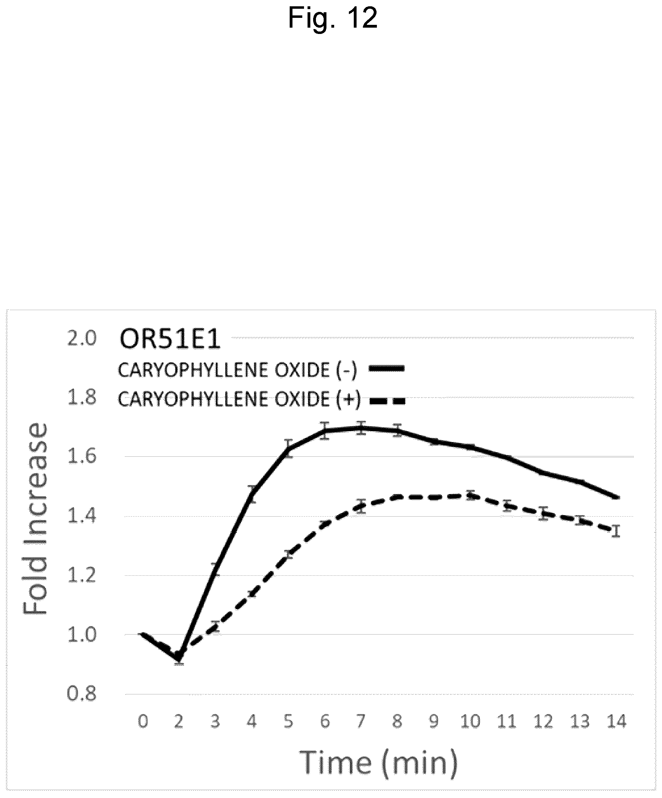

In the above luciferase assay, mixtures of hexanal and any of hexanal odor-suppressing materials: specifically, menthyl lactate, nootkatone, or beta-caryophyllene oxide, were used as samples and the changes in the fold increase values over time were determined in tests without the hexanal odor-suppressing material and in tests with the hexanal odor-suppressing material. The results are shown in Figures 7-12. The hexanal odor-suppressing materials (menthyl lactate, nootkatone, and beta-caryophyllene oxide) showed the effects in suppressing the responses of hexanal olfactory receptors OR2C1, OR2J2, OR2J3, OR5K1, OR5P3 and OR51E1 (Figures 7-12). These results indicate that the known hexanal odor-suppressing materials, represented by menthyl lactate, nootkatone, and beta-caryophyllene oxide, suppress the hexanal odor by suppressing at least one olfactory receptor of the group of hexanal olfactory receptors OR2C1, OR2J2, OR2J3, OR5K1, OR5P3 and OR51E1.

### INDUSTRIAL APPLICABILITY

The present invention can provide a method for screening a candidate substance for a hexanal odor-suppressing material that can inhibit the binding between hexanal and olfactory receptors OR2C1, OR2J2, OR2J3, OR5K1, OR5P3, and OR51E1.

## Claims

1. A method for screening a candidate substance for a hexanal odor-suppressing material, comprising:
adding a test substance and hexanal to at least one olfactory receptor polypeptide selected from the group consisting of olfactory receptors OR2C1, OR2J2, OR2J3, OR5K1, OR5P3, and OR51E1, and polypeptides that contain an amino acid sequence having 80% or more identity to the amino acid sequence of any of said olfactory receptors and that show responsiveness to hexanal;
measuring the response of the olfactory receptor polypeptide to hexanal; and
identifying, based on the measured response, the test substance which suppresses the response of the olfactory receptor polypeptide as a candidate substance for a hexanal odor-suppressing material.

2. The method according to claim 1, wherein the hexanal odor is an odor caused by hexanal or an odor containing at least some of said odor, which, in some cases, is an odor that is unpleasant to humans, and in some cases, is one or more of body odor, sweat odor, axillary odor, scalp odor, half-dried laundry odor, bathroom odor, nursing care odor, and deterioration odor of soybean or dairy products.

3. The method according to claim 1 or 2, comprising measuring the response of the olfactory receptor polypeptide to hexanal on cells isolated from a living body expressing the olfactory receptor or on cells artificially expressing the olfactory receptor by genetic engineering.

4. The method according to claim 1 or 2, wherein the response of the olfactory receptor polypeptide is measured by reporter assay or calcium imaging.
